(19) Europäisches Patentamt
European Patent Office
Office européen des brevets

(11) Veröffentlichungsnummer: **0 002 046**
**B1**

(12) # EUROPÄISCHE PATENTSCHRIFT

(45) Veröffentlichungstag der Patentschrift: **10.02.82**

(21) Anmeldenummer: **78101359.4**

(22) Anmeldetag: **13.11.78**

(51) Int. Cl.³: **C 07 H 15/04,**
C 07 D 493/04,
C 07 D 303/14,
C 07 H 19/00

(54) **Verfahren zur Herstellung von (1S,2S,6R)-2-Methyl-3,7-dioxabicyclo(4.1.0)heptan-4-Ol und dessen Methylether.**

(30) Priorität: **14.11.77 US 850982**

(43) Veröffentlichungstag der Anmeldung:
**30.05.79 Patentblatt 79/11**

(45) Bekanntmachung des Hinweises auf die Patenterteilung:
**10.02.82 Patentblatt 82/6**

(84) Benannte Vertragsstaaten:
**BE CH DE FR GB NL**

(56) Entgegenhaltungen:
**CHEMICAL COMMUNICATIONS, 1967, 973—975**

**CHEMICAL ABSTRACTS, Vol. 88, Nr. 191269u, 1978, Columbus, Ohio, USA, BOIVIN JEAN et al. "Synthesis of benzylidene acetals of methyl-2-deoxy-β-1-fucoside and methyl-α-D-digitoxoside," Seite 810**

(73) Patentinhaber: **F. HOFFMANN-LA ROCHE & CO. Aktiengesellschaft
CH-4002 Basel (CH)**

(72) Erfinder: **Grethe, Guenter
3 Andover Drive
North Caldwell, N.J. (US)**
Erfinder: **Uskokovic, Milan Radoje
253 Highland Avenue
Upper Montclair, N.J. (US)**
Erfinder: **Sereno, John
16 Parkway Drive
Pine Brook, N.J. (US)**

(74) Vertreter: **Lederer, Franz, Dr. et al,
Patentanwälte Lederer, Meyer Lucile-Grahn-Strasse 22
D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 002 046 B1**

Verfahren zur Herstellung von (1S,2S,6R)-2-Methyl-3,7-dioxabicyclo[4.1.0]heptan-4-ol und dessen Methylether.

Die vorliegende Erfindung betrifft ein Verfahren zur Herstellung von (1S,2S,6R) - 2 - Methyl - 3,7 - dioxabicyclo[4.1.0] - heptan - 4 - ol und dessen Methylether, (1S,2S,6R) - 4 - Methoxy - 2 - methyl - 3,7 - dioxabicyclo[4.1.0]heptan, die Zwischenprodukte für die Herstellung von L-Daunosamin sind.

L-Daunosamin (3 - Amino - 2,3,6 - trideoxy - L - lyxo - hexose) ist die Aminozucker-Komponente der Anthracyclin-Antibiotika Daunomycin und Adriamycin (Carbohydrate Research *44* (1975), 227—240), die eine bedeutende Rolle in der Antitumor-Therapie spielen. Synthesen für L-Daunosamin wurden beschrieben von Marsh et al. (Chem. Comm. *1967*, 973—975), ausgehend von dem teuren Zucker L-Rhamnose (nur geringe Gesamtausbeute), von Horton et al. (Carbohydrate Research *44* (1975), 227—240), ausgehend von D-Mannose (Gesamtausbeute 40%) und von Yamaguchi et al. (Carbohydrate Research *59* (1977), 343—350), ausgehend von dem Zuckerderivat 3 - Azido - 2S - benzyl - 4,6 - O - benzyliden - 3 - deoxy - 2 - thio - $\alpha$ - D - altropyranosid (nur geringe Gesamtausbeute).

Gemäss vorliegender Erfindung wird das bekannte (1S,2S,6R) - 4 - Methoxy - 2 - methyl - 3,7 - dioxabicyclo[4.1.0]heptan der Formel

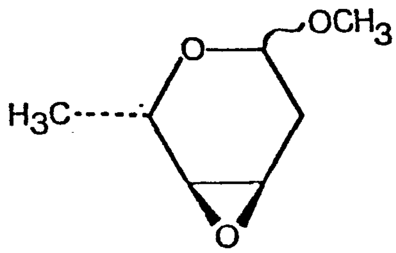

(VI)

als 4R- oder 4S-Anomer oder als Anomerengemisch, bzw. das noch neue (1S,2S,6R) - 2 - Methyl - 3,7 - dioxabicyclo[4.1.0] - heptan - 4 - ol der Formel

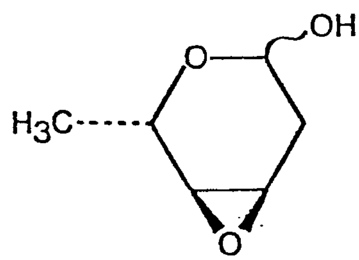

(V)

als 4R- oder 4S-Anomer oder als Anomerengemisch in einer Mehrstufenreaktion aus (1S,2S) - 2 - Methyl - 3 - cyclopenten - 1 - ol hergestellt. Die Reaktionsfolge ist dadurch gekennzeichnet, dass man (1S,2S) - 2 - Methyl - 3 - cyclopenten - 1 - ol epoxydiert zu (1S,2S,3S,5R) - 2 - Methyl - 6 - oxabicyclo[3.1.0]hexan - 3 - ol der Formel

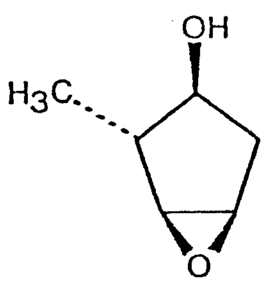

(II)

die Verbindung II durch Oxydation in (1S,2S,5R) - 2 - Methyl - 6 - oxabicyclo[3.1.0]hexan - 3 - on der Formel

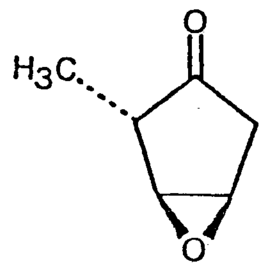

(III)

überführt, Verbindung III einer Baeyer-Villiger Oxidation zu (1S,2S,6R) - 2 - Methyl - 3,7 - dioxobicyclo[4.1.0]heptan - 4 - on der Formel

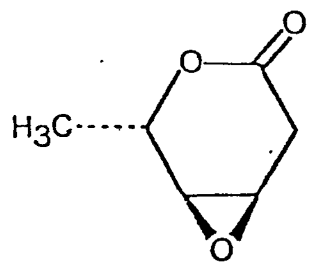

(IV)

unterwirft und Verbindung !V zur entsprechenden 4-Hydroxyverbindung reduziert, das 4R/4S-Anomerengemisch gewünschtenfalls auftrennt und gewünschtenfalls racemisches, 4R- oder 4S-(1S,2S,6R) - 2 - Methyl - 3,7 - dioxabicyclo[4.1.0]heptan - 4 - ol der Formel V durch Behandlung mit Methanol in Gegenwart eines sauren Katalysators methyliert und gewünschtenfalls ein erhaltenes Anomerengemisch auftrennt.

Die Reaktionssequenz ist im folgenden Schema dargestellt.

Reaktionsschema:

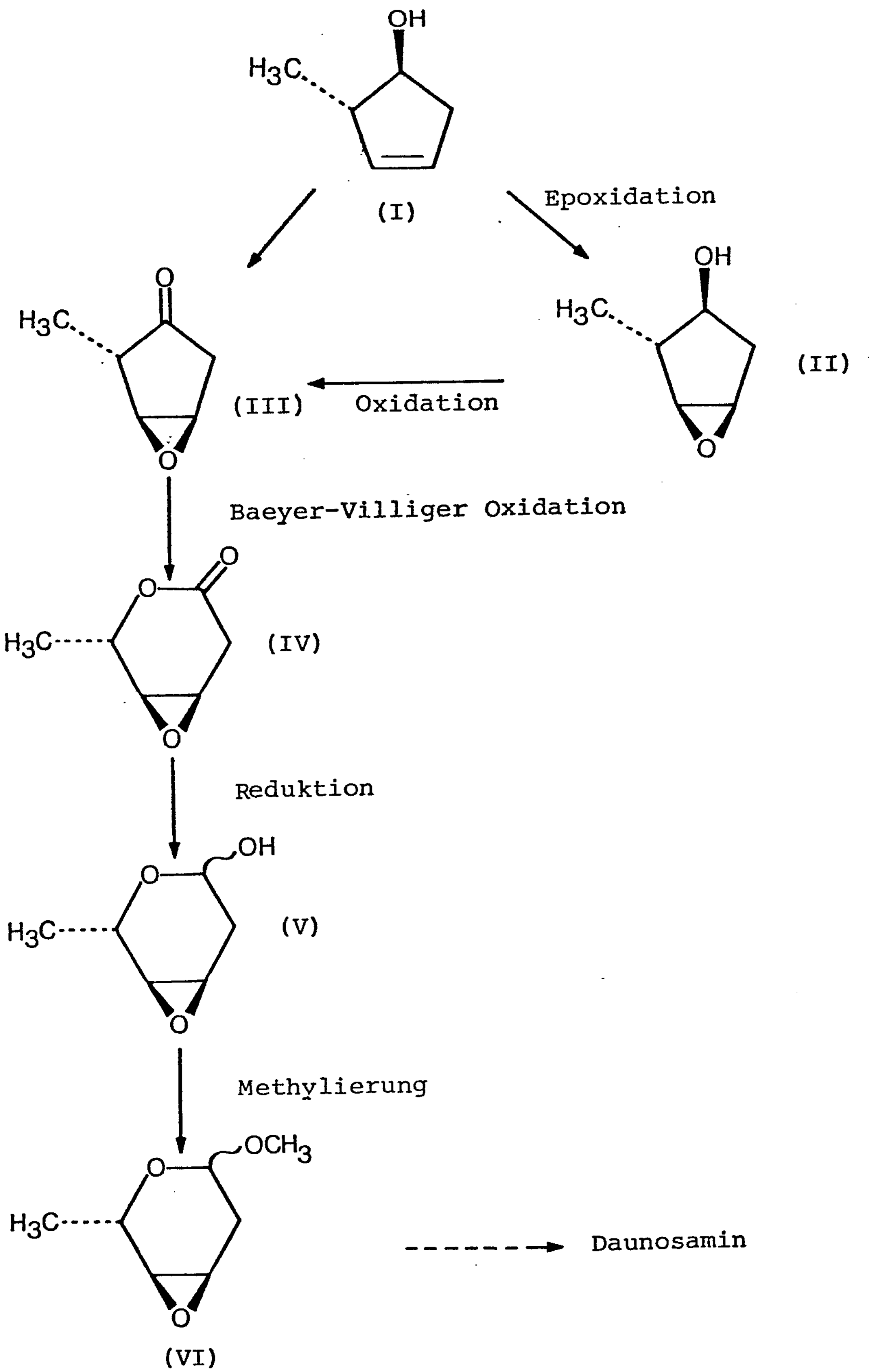

Die angegebenen Umsetzungen können in an sich bekannter Weise erfolgen. So kann die Epoxidation der Verbindung I unter Verwendung einer aliphatischen oder aromatischen Persäure, z.B. Peressigsäure, Perameisensäure, Trifluorperessigsäure, Permaleinsäure, Perbenzoesäure, Monoperphthalsäure, o-Sulfoperbenzosäure, p-Nitroperbenzoesäure und m-Chlorperbenzoesäure, wobei die letztere bevorzugt ist, mit Wasserstoffperoxid in saurem oder alkalischem Medium oder mit tert.-Butylhydroperoxid in alkalischem Medium erfolgen.

Die Reaktion kann in inerten, organischen Lösungsmitteln wie Diäthyläther, Chloroform, Tetrachlorkohlenstoff, Dichloräthan, Chloräthan, Benzol, Benzonitril, Acetonitril, Aethylacetat, Aceton, Pyridin oder, vorzugsweise, Methylenchlorid, oder aber in zweisphasigen Systemen, die aus den oben genannten Lösungsmitteln und einem wässrigen alkalischen Medium bestehen, erfolgen.

Als Puffersubstanzen eignen sich Natrium-, Kalium-, Calcium- oder Magnesiumformiat, -acetat oder -butyrate, Natriumdihydrogenphosphat, Dinatriumhydrogenphosphat oder Natrium-, Kalium-, Calcium- oder Magnesiumcarbonat, wobei Natriumcarbonat bevorzugt ist.

Das erhaltene (1S, 2S, 3S, 5R) - 2 - Methyl - 6 - oxabicyclo[3.1.0]hexan - 3 - ol wird anschliessend oxidiert unter Verwendung eines Oxidationsmittels wie Natrium- oder Kaliumdichromat in Schwefelsäure, Chromtrioxid in Essigsäure, Schwefelsäure oder Pyridin oder einer der oben genannten Perbenzoesäuren in Gegenwart von 2,2,6,6-Tetramethylpiperidin. Bevorzugt ist Chromtrioxid in wässriger Schwefelsäure (Jones Reagens). Geeignete Lösungsmittel für diese Reaktionen sind inerte organische Lösungsmittel wie Aceton, tert.-Butanol, Pyridin, Acetonitril, Dimethylformamid, Eisessig, Petroläther, Benzol, Diäthyläther und Tetrachlorkohlenstoff, wobei Aceton bevorzugt ist.

Das erhaltene (1S, 2S, 5R) - 2 - Methyl - 6 - oxabicyclo[3.1.0]hexan - 3 - on wird dann in einer Baeyer-Villiger Reaktion weiteroxidiert unter Verwendung einer der oben bereits genannten aliphatischen oder aromatischen Persäuren. Als geeignetes Oxidationsmittel kommt auch $H_2O_2$ in Schwefelsäure oder Bortrifluorid in Frage. Bevorzugt als Oxidationsmittel ist m-Chlorperbenzoesäure. Die Umsetzung wird geeigneterweise in einem inerten organischen Lösungsmittel wie Aethylacetat, Methylenchlorid oder Chloroform durchgeführt. Als Puffersubstanzen können die bereits oben genannten, vorzugsweise Natriumbicarbonat, verwendet werden.

Die Reduktion des erhaltenen (1S,2S,6R) - 2 - Methyl - 3,7 - dioxabicyclo[4.1.0]heptan - 4 - ons erfolgt mit einem Reduktionsmittel wie Diisobutylaluminiumhydrid, Diboran, Natrium- oder Lithiumtriäthoxyaluminiumhydrid, Natrium - bis - [2 - methoxyäthoxy] - aluminiumhydrid oder Tri-t-butoxyaluminiumhydrid, wobei Diisobutylaluminiumhydrid bevorzugt ist. Geeignete Lösungsmittel sind aromatische Kohlenwasserstoffe wie Toluol, Benzol und Xylol oder Aether wie Dioxan, Bis - (2 - methoxy) - äthyläther und Tetrahydrofuran, wobei Toluol bevorzugt ist. Das erhaltene Anomerengemisch kann in an sich bekannter Weise aufgetrennt werden.

In einer Reaktionsvariante kann die Verbindung III direkt aus (1S,2S) - 2 - Methyl - 3 - cyclopent - 1 - ol durch Umsetzung der Verbindung I mit einer der oben erwähnten aliphatischen oder aromatischen Persäuren, vorzugsweise mit m-Chlorperbenzosäure, und anschliessende in-situ-Zugabe von 2,2,6,6-Tetramethylpiperidin unter Verwendung der für die Reaktion I → II angegebenen inerten organischen Lösungsmittel hergestellt werden.

Für die Methylierung des (1S,2S,6R) - 2 - Methyl - 3,7 - dioxabicyclo[4.1.0]heptan - 4 - ols kommen als saure Katalysatoren organische oder anorganische Säuren, z.B. Lewis-Säuren, vorzugsweise $BF_3$, sowie Kationenaustauscherharze in Frage.

(1S,2S,6R) - 4 - Methoxy - 2 - methyl - 3,7 - dioxabicyclo[4.1.0]heptan kann in Daunosamin und weiter in Daunomycin nach aus der Literatur bekannten Methoden übergeführt werden (Chem. Commun. *1967*, 973—975 und J. Med. Chem. *17* (1974), 659—660).

Die vorliegende Erfindung eröffnet einen für die Totalsynthese des Daunomycins interessanten stereospezifischen Zugang zum Daunosamin aus einem leicht zugänglichen Ausgangsmaterial, das nicht der Zuckerreihe angehört. Das (1S,2S) - 2 - Methyl - 3 - cyclopenten - 1 - ol (I) kann aus 5 - Methylcyclopentadien nach einer publizierten Methode (J. Am. Chem. Soc. *95*, 532—540 [1973]) hergestellt werden.

## Beispiel 1

Zu einem Gemisch aus 27,2 g (1S,2S) - 2 - Methyl - 3 - cyclopenten - 1 - ol und 50 g Natriumbicarbonat in 400 ml wasserfreiem Methylenchlorid wurde unter Rühren bei 0°C innerhalb von 2 Stunden eine Lösung von 58,3 g 85%iger m-Chlorperbenzoesäure in einem Liter wasserfreiem Methylenchlorid gegeben. Nach beendeter Zugabe wurde weitere 2 Stunden bei 0°C gerührt und dann 38 g Kaliumcarbonat sowie 60 ml einer gesättigten wässrigen Lösung von Kaliumcarbonat zugesetzt. Der Niederschlag wurde durch Filtration abgetrennt und sorgfältig mit Methylenchlorid gewaschen. Das Filtrat wurde unter vermindertem Druch eingeengtund lieferte nach Destillation 21,8 g (1S,2S,3S,5R) - 2 - Methyl - 6 - oxabicyclo[3.1.0]hexan - 3 - ol; $Kp._{22}$ 72—75°C; $[\alpha]_D^{25}$ +43,35° (c, 1.169, Methanol).

Eine Lösung von 21,7 g (1S,2S,3S,5R) - 2 - Methyl - 6 - oxabicyclo[3.1.0]hexan - 3 - ol in 450 ml trockenem Aceton wurde bei 0°C innerhalb von 8 Minuten mit 90 ml 1N Jones Reagens versetzt. Die Suspension wurde 8 Minuten gerührt und anschliessend in 1 Liter Eiswasser gegossen. Das Gemisch wurde 5 × mit 250 ml Methylenchlorid extrahiert, die vereinigten Extrakte wurden

nacheinander mit gesättigter wässriger Bicarbonat- und Kochsalzlösung gewaschen, über Magnesiumsulfat getrocknet und eingedampft zu rohem, flüssigem (1S,2S,5R) - 2 - Methyl - 6 - oxabicyclo[3.1.0]hexan - 3 - on. Das Rohmaterial aus mehreren Ansätzen wurde über eine Vigreux Kolonne destilliert und lieferte 37 g reines (1S,2S,5R) - 2 - Methyl - 6 - oxabicyclo[3.1.0]hexan - 3 - on; Kp.$_8$ 60—66°C; $[\alpha]_D^{25} = +77$°C (c 1.10, Methanol).

Eine Lösung von 5,6 g (1S,2S,5R)-2-Methyl-6-oxabicyclo[3.1.0]hexan-3-on in 400 ml wasserfreiem Methylenchlorid wurde mit 15 g Natriumbicarbonat und 12,9 g 85%iger m-Chlorperbenzoesäure versetzt. Die Suspension wurde 72 Stunden bei Raumtemperatur gerührt und anschliessend durch Diatomeenerde filtriert. Das Filtrat wurde unter vers mindertem Druck zur Trockene eingeengt, der Rückstand mit 40 ml Methylenchlorid behandelt und filtriert. Das Filtrat wurde zweimal mit gesättigter wässriger Natriumbicarbonatlösung gewaschen, über Magnesiumsulfat getrocknet und unter vermindertem Druck zur Trockene eingeengt. Durch Erhitzen auf 70°C (0.1 mmHg) wurden 0,95 g flüchtige Bestandteile entfernt. Der ölige Rückstand kristallisierte langsam beim Stehen und lieferte (1S,2S,6R) - 2 - Methyl - 3,7 - dioxabicyclo[4.1.0] - heptan - 4 - on; F. 51—52°C. Ausbeute nack Umkristallisation aus 12 ml Aether: 5,05 g. Nach mehreren Umkristallisationen aus Aether wurde analytisch reines (1S,2S,6R) - 2 - Methyl - 3,7 - dioxabicyclo[4.1.0]heptan - 4 - on erhalten; F. 52,5—53,5°C; $[_D^{25} = +147,26$° (c 0.995, Methanol).

Eine auf —75°C gekühlte Lösung von 2,58 g (1S,2S,6R) - 2 - Methyl - 3,7 - dioxabicyclo[4.1.0]heptan - 4 - on in 300 ml wasserfreiem Toluol wurde unter Argon mit 14 ml einer 1,75 M Lösung von Diisobutylaluminiumhydrid in Toluol versetzt. Das Gemisch wurde weitere 2 Stunden bei —75°C gerührt und dann schnell in ein Gemisch aus 260 g Kieselgel in Toluol gegossen. Es wurde filtriert und mit 3 l Methylenchlorid/Methanol (9:1, v/v) gewaschen. Das Filtrat wurde unter vermindertem Druck eingeengt und der erhaltene ölige Rückstand in einem Kugelrohrdestillationsapparat destilliert. Es wurden 2,03 g (1S,2S,6R- - 2 - Methyl - 3,7 - dioxabicyclo[4.1.0] - heptan - 4 - ol, Kp.$_{0,1}$ 90°C, $[\alpha]_D^{25} = +25,16$° (c 1.013, Methanol), erhalten.

Zu einer Lösung von 8,8 g (1S,2S,6R) - 2 - Methyl - 3,7 - dioxabicyclo[4.1.0]heptan - 4 - ol in 150 ml wasserfreiem Methanol wurden unter Argonatmosphäre und Rühren bei 35°C 0,45 ml einer 8,8%igen (w/v), frisch hergestellten Lösung von Bortrifluorid in wasserfreiem Methanol gegeben. Das Gemisch wurde 2 Stunden bei 35°C gerührt und dann mit 2,5 g Natriumbicarbonat versetzt und weitere 15 Minuten bei Raumtemperatur gerührt. Es wurde filtriert, der Rückstand 3 × mit 10 ml Methylenchlorid gewaschen und das Filtrat unter vermindertem Druck eingeengt. Der halbfeste Rückstand wurde mit Methylenchlorid behandelt, Feststoffe wurden durch Filtration abgetrennt und das Filtrat wurde unter vermindertem Druck eingeengt. Der Rückstand wurde durch einen Kugelrohrdestillationsapparat bei 90°C (Ofentemperatur) und einem Druck von 0,1 mmHg destilliert und lieferte 8,3 g des rohen Anomerengemisches von (1S,2S,6R) - 4 - Methoxy - 2 - methyl - 3,7 - dioxabicyclo[4.1.0]heptan in Form einer hellgelben Flüssigkeit.

Beispiel 2

Auf einem anderen Weg wurde (1S,2S,5R) - 2 - Methyl - 6 - oxabicyclo[3.1.0]hexan - 3 - on hergestellt durch Zusatz einer Lösung von 6,20 g 85%iger m-Chlorperbenzoesäure in 100 ml wasserfreiem Methylenchlorid innerhalb von 30 Minuten unter Rühren zu einem Gemisch aus 2,28 g (1S,2S,3S,5R) - 2 - Methyl - 6 - oxabicyclo[3.1.0]hexan - 3 - ol, 1 ml einer 0,2 M Lösung von 2,2,6,6-Tetramethylpiperidinhydrochlorid in einem 1%igem Gemisch aus Aethanol und Methylenchlorid und 20 ml Methylenchlorid. Das Reaktionsgemisch wurde eine Stunde bei 0°C gerührt. Eine Hälfte des Gemisches wurde 2 × mit 25 ml 1N wässriger Natriumhydroxidlösung und anschliessend mit Kochsalzlösung gewaschen. Die organische Phase wurde über Magnesiumsulfat getrocknet, filtriert und unter vermindertem Druck zur Trockene eingeengt. Der Rückstand wurd durch einen Kurzwegdestillationsapparat destilliert (80°C, 20 mmHg) und lieferte 0,452 g flüssiges (1S,2S,5R) - 2 - Methyl - 6 - oxabicyclo[3.1.0]hexan - 3 - on.

Beispiel 3

Auf einem anderen Weg wurde (1S,2S,5R) - 2 - Methyl - 6 - oxabicyclo[3.1.0]hexan - 3 - on aus (1S,2S) - 2 - Methyl - 3 - cyclopenten - 1 - ol in folgender Weise erhalten:

Zu einer Lösung von 1,96 g (1S,2S) - 2 - Methyl - 3 - cyclopenten - 1 - ol in 20 ml wasserfreiem Methylenchlorid wurde unter Rühren innerhalb von 20 Minuten bei 0°C und unter Argonatmosphäre eine Lösung von 4,44 g 85%iger m-Chlorperbenzoessäure gegeben. Das Gemisch wurde weitere 2 Stunden bei 0°C gerührt. Der entstandene Niederschlag wurde durch Filtration entfernt und der Rückstand mit Methylenchlorid gewaschen. Dem Filtrat wurden bei 0°C 4,5 g 85%ige m-Chlorperbenzoesäure und 1 ml einer 0.2 M Lösung von 2,2,6,6-Tetramethylpiperidinhydrochlorid in einem 1%igen Gemisch aus Aethanol und Methylenchlorid zugesetzt. Das Gemisch wurde 30 Minuten bei 0°C und 2 Stunden bei 35°C gerührt. Der Niederschlag wurde abfiltriert und das Filtrat 2 × mit 20 ml gesättigter wässriger Natriumbicarbonatlösung und anschliessend mit Kochsalzlösung gewaschen. Die organische Phase wurde über Magnesiumsulfat getrocknet und unter vermindertem Druck zur Trockene eingeengt. Destillation des Rückstands in einem Kugelrohrapparat (80°C, 20 mmHg) lieferte 0,533 g (1S,2S,5R) - 2 - Methyl - 6 - oxabicyclo[3.1.0]hexan - 3 - on.

**Patentansprüche**

1. Verfahren zur Herstellung von (1S,2S,6R) - 2 - Methyl - 3,7 - dioxabicyclo[4.1.0]heptan - 4 - ol der Formel

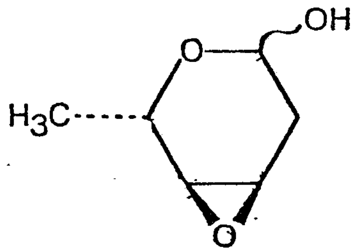

(V)

als 4R- oder 4S-Anomer oder als Anomerengemisch und des entsprechenden Methylethers der Formel

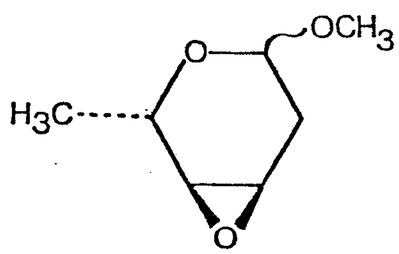

(VI)

als 4R- oder 4S-Anomer oder als Anomerengemisch, dadurch gekennzeichnet, dass man (1S,2S) - 2 - Methyl - 3 - cyclo - penten - 1 - ol epoxydiert zu (1S,2S,3S,5R) - 2 - Methyl - 6 - oxabicyclo[3.1.0]hexan - 3 - ol der Formel

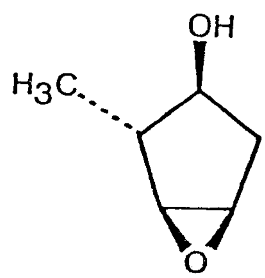

(II)

die Verbindung II durch Oxydation in (1S,2S,5R) - 2 - Methyl - 6 - oxabicyclo[3.1.0]hexan-3-on der Formel

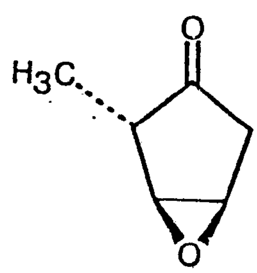

(III)

überführt. Verbindung III einer Baeyer-Villiger Oxidation zu (1S,2S,6R) - 2 - Methyl - 3,7 - dioxabicyclo[4.1.0]heptan - 4 - on der Formel

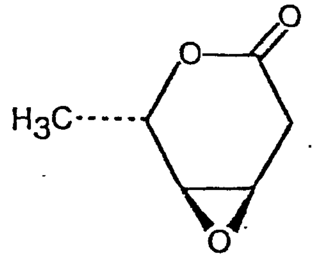

(IV)

unterwirft und Verbindung IV zur entsprechenden 4-Hydroxyverbindung reduziert, das 4R/4S-Anomerengemisch gewünschtenfalls auftrennt und gewünschtenfalls racemisches, 4R- oder 4S - (1S,2S,6R) - 2 - Methyl - 3,7 - dioxabicyclo[4.1.0]heptan - 4 - ol der Formel V durch Behandlung mit Methanol in Gegenwart eines sauren Katalysators methyliert und gewünschtenfallsein erhaltenes Anomerengemisch auftrennt.

2. Verfahren nach Anspruch 1, dadurch gekennzeichnet, dass das (1S,2R,5R) - 2 - Methyl - 6 - oxabicyclo[3.1.0]hexan - 3 - on der Formel III aus (1S,2S) - 2 - Methyl - 3 - cyclopenten - 1 - ol durch Umsetzung mit einer organischen oder anorganischen Persäure und anschliessende in-situ-Zugabe von 2,2,6,6-Tetramethylpiperidin hergestellt wird.

**Claims**

1. A process for the manufacture of (1S,2S,6R) - 2 - methyl - 3,7 - dioxabicyclo[4.1.0]heptan - 4 - ol of the formula

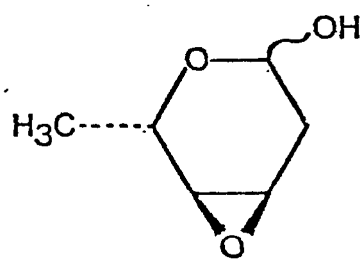

(V)

as the 4R- or 4S-anomer or as the anomer mixture and of the corresponding methyl ether of the formula

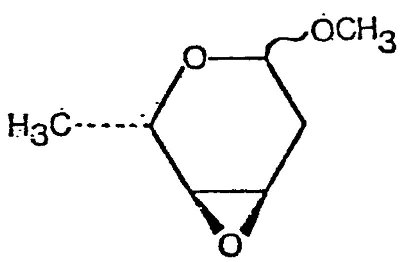

(VI)

as the 4R- or 4S-anomer or as the anomer mixture, characterized by epoxidizing (1S,2S) - 2 - methyl - 3 - cyclo - penten - 1 - ol to give (1S,2S,3S,5R) - 2 - methyl - 6 - oxabicyclo[3.1.0]hexan - 3 - ol of the formula

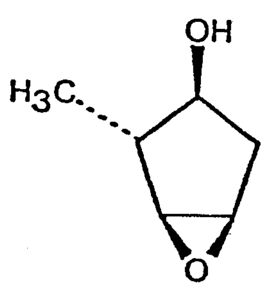

(II)

converting the compound II by oxidation into (1S,2S,5R) - 2 - methyl - 6 - oxabicyclo[3.1.0]hexan - 3 - one of the formula

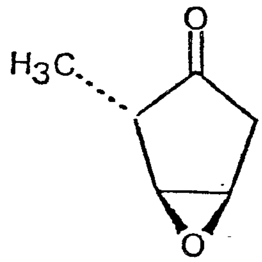

(III)

subjecting compound III to a Baeyer-Villiger oxidation to give (1S,2S,6R) - 2 - methyl - 3,7 - dioxabicyclo[4.1.0]heptan - 4 - one of the formula

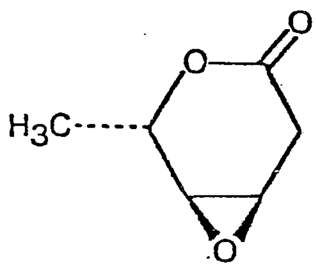

(IV)

and reducing compound IV to the corresponding 4-hydroxy compound, separating the 4R/4S-anomer mixture if desired and, if desired, methylating racemic, 4R- or 4S - (1S,2S,6R- - 2 - methyl - 3,7 - dioxabicyclo[4.1.0]heptan - 4 - ol of formula V by treatment with methanol in the presence of an acidic catalyst and, if desired, separating an anomer mixture obtained.

2. A process according to claim 1, characterized in that the (1S,2S,5R) - 2 - methyl - 6 - oxabicyclo[3.1.0]hexan - 3 - one of formula III is manufactured from (1S,2S) - 2 - methyl - 3 - cyclopenten - 1 - ol by reaction with an organic or inorganic peracid and subsequent in situ addition of 2,2,6,6-tetramethylpiperidine.

**Revendications**

1. Procédé de préparation de (1S,2S,6R) - 2 - méthyl - 3,7 - dioxabicyclo[4.1.0]heptan - 4 - ol der formule

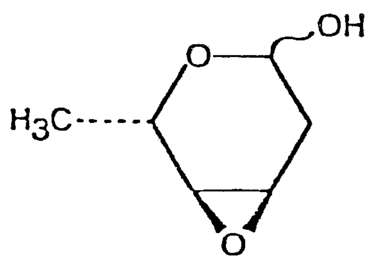

(V)

sous forme d'anomère 4R ou 4S ou sous forme de mélange d'anomères et du méthyléther correspondant de formule

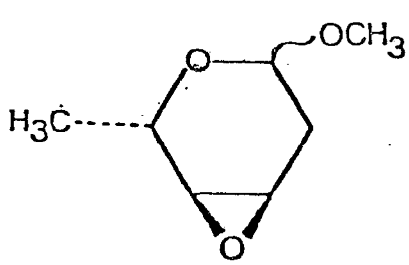

(VI)

sous forme d'anomère 4R ou 4S, caractérisé en ce qu'on époxyde le (1S,2S) - 2 - méthyl - 3 - cyclo - pentén - 1 - ol en (1S,2S,3S,5R) - 2 - méthyl - 6 - oxabicyclo[3.1.0]hexan - 3 - ol de formule

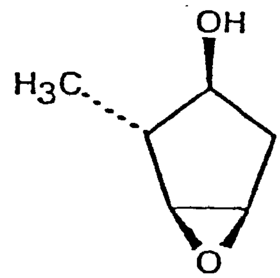

(II)

en ce qu'on transforme le composé II par oxydation en (1S,2S,5R) - 2 - méthyl - 6 - oxabicyclo[3.1.0]hexan - 3 - one de formule

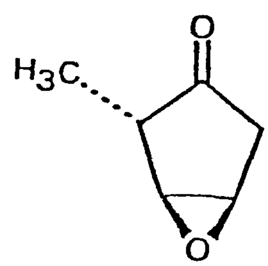

(III)

en ce qu'on soumet le composé III à une oxydation de Baeyer-Villeger pour donner la (1S,2S,6R) - 2 - méthyl - 3,7 - dioxabicyclo[4.1.0]heptan - 4 - one de formule

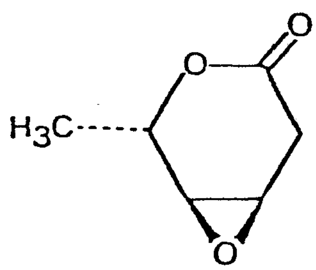

(IV)

et en ce qu'on réduit le composé IV pour donner le composé 4-hydroxy correspondant, éventuellement en ce qu'on 4-hydroxy correspondant, éventuellement en ce qu'on dédouble le mélange d'isomères 4R/4S et si on le désire en ce qu'on méthyle le (1S,2S,6R) - 2 - méthyl - 3,7 - dioxabicyclo[4.1.0]heptan - 4 - ol racémique, 4R ou 4S de formule V par traitement avec du méthanol en présence d'un catalyseur acide et éventuellement en ce qu'on dédouble un mélange d'isomères obtenu.

2. Procédé selon la revendication 1, caractérisé en ce qu'on prépare la (1S,2S,5R) - 2 - méthyl - 6 - oxabicyclo[3.1.0]hexan - 3 - one de formule III à partir du (1S,2S) - 2 - méthyl - 3 - cyclopentén - 1 - ol par réaction avec un peracide organique ou inorganique puis par addition in situ de 2,2,6,6 - tetraméthylpipéridine.